# EUROPEAN PATENT APPLICATION

(11) **EP 4 470 541 A1**
(43) Date of publication of application: **04.12.2024**
(21) Application number: 23747382.2
(22) Date of filing: 27.01.2023
(51) Int. Cl.: A61K 31/541, A61P 43/00, A61P 39/00, A61P 29/00

(54) **OVARIAN ANTI-AGING PHARMACEUTICAL COMPOSITION AND USE THEREOF**

(30) Priority: 27.01.2022 KR 20220012507; 27.01.2022 KR 20220012508
(71) Applicant: MitoImmune Therapeutics Inc., Seoul 06123 (KR)
(72) Inventor: KIM, Soon Ha, Seoul 06123 (KR); LEE, Jae Ho, Seoul 06123 (KR); KIM, Yu Jin, Seoul 06123 (KR)
(74) Representative: Plasseraud IP
(86) International application number: PCT/KR2023/001264
(87) International publication number: WO 2023/146341

(57) **Abstract**

The present invention relate to: an ovarian anti-aging pharmaceutical composition comprising, as an active ingredient, a compound of chemical formula 1 or a pharmaceutically acceptable salt thereof; an ovarian anti-aging method comprising same; and a pharmaceutical composition for preventing or treating diseases related to ovarian aging or dysfunction, wherein the compound of chemical formula 1 may exhibit the effects of restoring ovarian function and inhibiting ovarian aging, and may be effectively used for preventing or treating diseases related to ovarian aging or dysfunction.

## Description

### [Technical Field]

The present invention relates to a pharmaceutical composition for anti-ovarian aging, which includes a compound of Chemical Formula 1 or a pharmaceutically acceptable salt thereof, a pharmaceutical composition for preventing or treating a disease related to the aging or dysfunction of ovaries, and a method for anti-ovarian aging.

### [Background Art]

Currently, as the reproductive age group of women increases, the infertility rate in women is increasing, and ovarian function plays an important role in successful pregnancy outcomes. However, there is no known treatment method for infertility associated with dysfunction of aging ovarian, which is manifested by a decline in of oocyte and embryo capacity (Cimadomo et al. 2018).

The development of ovarian follicles goes through a very complex process regulated by several factors in gonadotrophin-independent and -dependent stages, and oocyte quality is related to the function of somatic cells in the ovaries, such as granulosa cells and cumulus cells. Among older women attempting in vitro fertilization (IVF), those with a successful history of normal childbirth may exhibit different types of granulosa cells and cumulus cells during the development of ovarian follicles, compared to those who have failed to conceive. Accordingly, to restore ovarian function, follicle development and the functions of not only oocytes but also cumulus cells and granulosa cells are important.

To date, the mechanism of ovarian aging or the decisive factor in infertility due to maternal aging has not been identified, and recently, several cellular and molecular characteristics caused by the progression of aging, such as telomere shortening, genome instability, decreased autophagy, stem cell exhaustion, and mitochondrial dysfunction were confirmed.

Advances in medicine and improved economic standards have led to an aging society, and as a result, the number of aging mothers, attempting to become pregnant at a later age, is increasing. However, due to aging, ovarian function gradually decreases, leading to infertility and subfertility cases, and an increase in diseases related to aged ovaries.

Many types of anti-aging agents are being developed to prevent the progression of aging and achieve rejuvenation. However, the development of treatments that can restore aged ovaries or inhibit ovarian aging is minimal.

### [Related Art]

### [Patent Document]

International Patent Publication No. WO2009/025478

### [Disclosure]

### [Technical Problem]

The present invention is directed to providing a material for recovering the function of aged ovaries and inhibiting ovarian aging.

The present invention is also directed to providing a pharmaceutical composition for anti-ovarian aging, which includes a compound of Chemical Formula 1 or a pharmaceutically acceptable salt thereof as an active ingredient, and a method for anti-ovarian aging using the above-mentioned component.

The present invention is also directed to providing a pharmaceutical composition for preventing or treating a disease related to the aging or dysfunction of ovaries, which includes the above-mentioned component, and a method of preventing or treating a disease related to the aging or dysfunction of ovaries, which includes the above-mentioned component.

### [Technical Solution]

The present inventors confirmed that a compound of Chemical Formula 1 or a pharmaceutically acceptable salt thereof can restore the function of aged ovaries, and thus completed the present invention.

Therefore, the present invention provides a pharmaceutical composition for anti-ovarian aging, which includes a compound of Chemical Formula 1 or a pharmaceutically acceptable salt thereof as an active ingredient.

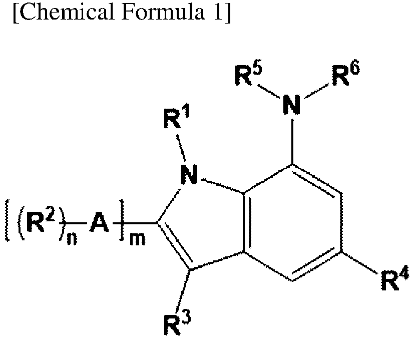

In this formula,
n may be an integer from 1 to 3,
m may be 0 or 1,
A may be phenyl,
R¹ may be hydrogen or C₁-C₆ alkyl,
R² may be hydrogen, halogen, C₁-C₆ alkoxy, -C₁₋C₆ alkylene-OH, -(CH₂)ₚCO₂R⁷, - NHR⁸, -N(H)S(O)₂R⁷, or -NHC(O)R⁷, wherein p is an integer from 0 to 3, R⁷ is hydrogen or C₁-C₃ alkyl, and R⁸ is C₁-C₃ alkylpiperidinyl or C₁-C₃ alkylsulfonyl,
R³ may be hydrogen, halogen, C₁-C₆ alkyl, phenyl, or -(CH₂)ₚ-heterocycle, in which the heterocycle is a 5- or 6-membered ring including 1 or 2 hetero atoms selected from S, N, and O atoms, wherein p is an integer from 0 to 3, provided that when m is 0, R³ is phenyl,
R⁴ may be halogen, C₁-C₆ alkyl, -C₁₋C₆ alkylene-OH, -O-phenyl, -(CH₂)ₚCO₂R⁷, -(CH₂)ₚ-heterocycle, in which the heterocycle is a 5- or 6-membered ring including 1 or 2 hetero atoms selected from S, N, and O atoms, or proline-N-carbonyl wherein p is an integer from 0 to 3, and R⁷ is the same as defined above,
R⁵ may be hydrogen or C₁-C₆ alkyl,
R⁶ may be C₁-C₆ alkyl, C₃-C₆ cycloalkyl, heterocycle, or -C₁-C₆ alkylene-heterocycle, in which the heterocycle is a 3- to 8-membered ring including 1 to 3 hetero atoms selected from S, N, and O atoms, and R⁶ may be substituted with C₁-C₆ alkylamine, -C₁₋C₆ alkylene-OH, or C₁-C₆ alkylsulfonyl.

The present invention also provides a method for anti-ovarian aging, which includes administering a compound of Chemical Formula 1 or a pharmaceutically acceptable salt thereof into a subject in need thereof.

The present invention also provides a pharmaceutical composition for preventing or treating a disease related to ovarian aging, which includes a compound of Chemical Formula 1 or a pharmaceutically acceptable salt thereof.

### [Advantageous Effects]

According to a composition or method of the present invention, when a compound of Chemical Formula 1 is treated, it can exhibit an effect of restoring the function of aged ovaries and inhibiting ovarian aging by increasing the functional activity of ovaries such as increasing oocytes and the development of ovarian follicles, and increasing the expression of specific genes for recovery from ovarian aging. Therefore, the compound of Chemical Formula 1 of the present invention can be effectively used in diseases related to ovarian dysfunction due to aging.

### [Description of Drawings]

FIG. 1A shows a set of histological images of mouse ovarian tissue stained with hematoxylin and eosin after treatment with a compound of Example and stimulation with a hormone according to Experimental Example 1.
FIGS. 1B and 1C show a set of images and a graph exhibiting the number of oocytes according to the presence or absence of treatment with the compound of Example at a germinal vesicle (GV) stage according to Experimental Example 1.
FIG. 2 is a table showing the effect of treatment with the compound of Example on fertilized 2 pronuclear cell (2PN) embryo development according to Experimental Example 2.
FIG. 3A is a graph showing the results of next generation sequencing (NGS) according to Experimental Example 3.
FIG. 3B is a graph showing gene expression levels associated with tumor necrosis factor superfamily cytokine production according to Experimental Example 3.
FIGS. 4A and 4B are images and a quantification graph showing the results of reverse transcription-polymerase chain reaction (RT-PCR) analysis for each gene according to Experimental Example 4.

### [Modes of the Invention]

Hereinafter, the present invention will be described in detail.

Meanwhile, each description and embodiment disclosed in the present invention may be applied to other descriptions and embodiments. That is, all combinations of the various elements disclosed in the present invention are interpreted as falling within the scope of the present invention. In addition, the scope of the present invention is not limited by the specific description described below.

When one part "includes" a certain component, unless particularly stated otherwise, other components may be further included, rather than excluded.

The present invention provides a composition for anti-ovarian aging, which includes a compound of Chemical Formula 1, or a pharmaceutically acceptable salt thereof as an active ingredient.

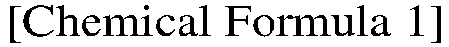

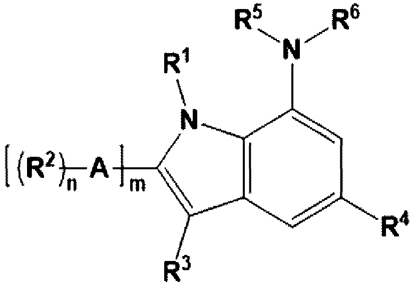

In this formula,
n may be an integer from 1 to 3,
m may be 0 or 1,
A may be phenyl,
R¹ may be hydrogen or C₁-C₆ alkyl,
R² may be hydrogen, halogen, C₁-C₆ alkoxy, -C₁₋C₆ alkylene-OH, -(CH₂)ₚCO₂R⁷, - NHR⁸, -N(H)S(O)₂R⁷, or -NHC(O)R⁷, wherein p is an integer from 0 to 3, R⁷ is hydrogen or C₁-C₃ alkyl, and R⁸ is C₁-C₃ alkylpiperidinyl or C₁-C₃ alkylsulfonyl,
R³ may be hydrogen, halogen, C₁-C₆ alkyl, phenyl, or -(CH₂)ₚ-heterocycle, in which the heterocycle is a 5- or 6-membered ring including 1 or 2 hetero atoms selected from S, N, and O atoms, wherein p is an integer from 0 to 3, provided that when m is 0, R³ is phenyl,
R⁴ may be halogen, C₁-C₆ alkyl, -C₁₋C₆ alkylene-OH, -O-phenyl, -(CH₂)ₚCO₂R⁷, -(CH₂)ₚ-heterocycle, in which the heterocycle is a 5- or 6-membered ring including 1 or 2 hetero atoms selected from S, N, and O atoms, or proline-N-carbonyl wherein p is an integer from 0 to 3, and R⁷ is the same as defined above,
R⁵ may be hydrogen or C₁-C₆ alkyl,
R⁶ may be C₁-C₆ alkyl, C₃-C₆ cycloalkyl, heterocycle, or -C₁-C₆ alkylene-heterocycle, in which the heterocycle is a 3- to 8-membered ring including 1 to 3 hetero atoms selected from S, N, and O atoms, and R⁶ may be substituted with C₁-C₆ alkylamine, -C₁₋C₆ alkylene-OH, or C₁-C₆ alkylsulfonyl.

The present invention confirmed an effect of restoring the function of aged ovaries by the compound of Chemical Formula 1 through the treatment of aged ovaries with the compound of Chemical Formula 1. Thus, the present invention identifies a novel use of the compound of Chemical Formula 1.

The compound of Chemical Formula 1 of the present invention may be used in the form of a pharmaceutically acceptable salt thereof. Particularly, the pharmaceutically acceptable salt may be an acid addition salt formed by a free acid. Here, the acid addition salt may be obtained from inorganic acids such as hydrochloric acid, nitric acid, phosphoric acid, sulfuric acid, hydrobromic acid, hydroiodic acid, nitrous acid or phosphorous acid, non-toxic organic acids such as aliphatic mono- and di-carboxylates, phenyl-substituted alkanoates, hydroxy alkanoates and alkane dioates, aromatic acids, aliphatic, and aromatic sulfonic acids, and organic acids such as trifluoroacetic acid, acetate, benzoic acid, citric acid, lactic acid, maleic acid, gluconic acid, methanesulfonic acid, 4-toluenesulfonic acid, tartaric acid, and fumaric acid. Types of such pharmaceutically acceptable salts may include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, and propionate.

The composition of the present invention may include not only the compound of Chemical Formula 1 and a pharmaceutically acceptable salt thereof, but also any salt, isomer, hydrate, and/or solvate, which can be prepared by a conventional method.

The "isomer" used herein may refer to compounds of the present invention, which have the same chemical formula or molecular formula, but are structurally or sterically different, or salts thereof. These isomers include structural isomers such as tautomers, R or S isomers with asymmetric carbon centers, geometric isomers (trans, cis), and optical isomers (enantiomers). All these isomers and mixtures thereof are also included within the scope of the present invention.

The "hydrate" used herein may mean a compound of the present invention, which includes a stoichiometric or non-stoichiometric amount of water, which is bound by a non-covalent intermolecular force, or a salt thereof. A hydrate of the compound represented by Chemical Formula 1 of the present invention may include a stoichiometric or non-stoichiometric amount of water, which is bound by a non-covalent intermolecular force. The hydrate may contain 1 eq or more, and preferably, 1 to 5 eq of water. Such a hydrate may be prepared by crystallizing a compound represented by Chemical Formula 1 of the present invention, an isomer thereof, or a pharmaceutically acceptable salt thereof from water or a water-containing solvent.

The "solvate" used herein may refer to a compound of the present invention, which includes a stoichiometric or non-stoichiometric amount of solvent, which is bound by a non-covalent intermolecular force, or a salt thereof. Preferred solvents therefor include solvents that are volatile, non-toxic, and/or suitable for administration to humans.

The term "alkyl" used herein refers to an aliphatic hydrocarbon radical. Alkyl may be "saturated alkyl" that does not include an alkenyl or alkynyl moiety, or "unsaturated alkyl" that includes at least one alkenyl or alkynyl moiety, and have, unless defined otherwise, 1 to 20 carbon atoms. Alkyl, alkenyl, and alkynyl may mean linear or branched acyclic hydrocarbons.

The term "alkylene" may mean a bivalent hydrocarbon group in which a radical is additionally formed from the alkyl, and include, for example, methylene, ethylene, propylene, butylene, and isobutylene, but the present invention is not limited thereto.

The term "alkoxy" refers to, unless defined otherwise, alkyl-oxy having 1 to 10 carbon atoms.

The term "cycloalkyl" refers to, unless defined otherwise, a saturated aliphatic 3- to 10-membered ring. Typical cycloalkyl groups include cyclopropyl, cyclobutyl, cyclopentyl, and cyclohexyl, but the present invention is not limited thereto.

The term "heterocycle" refers to, unless defined otherwise, a 3- to 10-membered ring, preferably, a 4- to 8-membered ring, and more preferably, a 5- to 6-membered ring, which includes 1 to 3 hetero atoms selected from the group consisting of N, O, and S, may be fused with benzo or C₃-C₈ cycloalkyl, is saturated, or has 1 or 2 double bonds. In addition, the "heterocycle" may be used interchangeably with the term "heterocyclyl." Examples of heterocycles may include pyrroline, pyrrolidine, imidazoline, imidazolidine, pyrazoline, pyrazolidine, pyran, piperidine, morpholine, thiomorpholine, piperazine, and hydrofuran, but the present invention is not limited thereto.

Other terms and abbreviations used herein, unless defined otherwise, can be interpreted as generally understood by those of ordinary skill in the art to which the present invention pertains.

In one embodiment of the present invention, in the compound of Chemical Formula 1,

R³ may be hydrogen, halogen, phenyl, or -(CH₂)ₚ-heterocycle in which the heterocycle is morpholino or piperazinonyl. Here, p is an integer of 0 or 1, provided that when m is 0, R³ may be phenyl.

In one embodiment of the present invention, in the compound of Chemical Formula 1,

R⁴ may be halogen, C₁-C₃ alkyl, -C₁₋C₃ alkylene-OH, -O-phenyl, -(CH₂)ₚCO₂-ethyl, (CH₂)ₚ-heterocycle in which the heterocycle is thiomorpholino, morpholino, piperazinonyl, or pyrrolidinyl, or proline-N-carbonyl, wherein p may be an integer of 0 or 1.

In one embodiment of the present invention, in the compound of Chemical Formula 1,
R⁵ may be hydrogen or C₁-C₃ alkyl,
R⁶ may be C₁-C₃ alkyl, C₃-C₆ cycloalkyl, heterocycle, or -C₁-C₃ alkylene-heterocycle, wherein the heterocycle is tetrahydro-2H-pyran, or piperidinyl. When R⁶ is heterocycle or - C₁-C₃ alkylene-heterocycle, the heterocycle may be substituted with C₁-C₆ alkylamine, -C₁₋C₆ alkylene-OH, or C₁-C₆ alkylsulfonyl.

In the present invention, examples of the compound of Chemical Formula 1 may include compounds 1 to 32 listed in Table 1 below, or pharmaceutically acceptable salts thereof.

**[Table 1]**

| No. | Structure | Name of Compound |
|---|---|---|
| 1 | 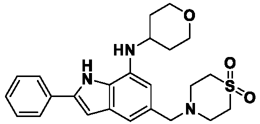 | 5-[(1,1-dioxido-4-thiomorpholinyl)methyl]-2-phenyl-N-(tetrahydro-2H-pyran-4-yl)-1H-indol-7-amine |
| 2 | 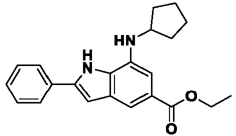 | ethyl 7-(cyclopenylamino)-2-phenyl-1H-indol-5-carboxylate |
| 3 | 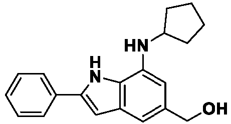 | (7-(cyclopenylamino)-2-phenyl-1H-indol-5-yl)methanol |
| 4 | 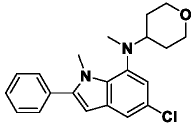 | 5-chloro-N,1-dimethyl-2-phenyl-N-(tetrahydro-2H-pyran-4-yl)-1H-indol-7-amine |
| 5 | 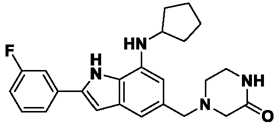 | 4-((7-(cyclopenylamino)-2-(3-fluorophenyl)-1H-indol-5-yl)methyl)piperazin-2-one |
| 6 | 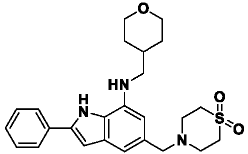 | 4-((2-phenyl-7-(((tetrahydro-2H-pyran-4-yl)methyl)amino)-1H-indol-5-yl)methyl)thiomorpholine 1,1-dioxide |
| 7 | 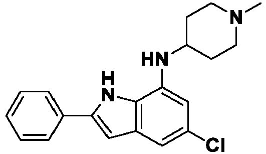 | 5-chloro-N-(1-methylpiperidin-4-yl)-2-phenyl-1H-indol-7-amine |
| 8 | 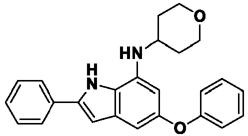 | 5-phenoxy-2-phenyl-N-(tetrahydro-2H-pyran-4-yl)-1H-indol-7-amine |
| 9 | 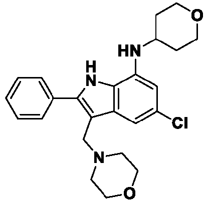 | 5-chloro-3-(morpholinomethyl)-2-phenyl-N-(tetrahydro-2H-pyran-4-yl)-1H-indol-7-amine |
| 10 | 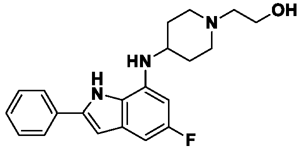 | 2-(4-((5-fluoro-2-phenyl-1H-indol-7-yl)amino)piperidin-1-yl)ethan-1-ol |
| 11 | 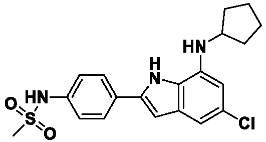 | N-(4-(5-chloro-7-(cyclopenylamino)-1H-indol-2-yl)phenyl)methanesulfonamide |
| 12 | 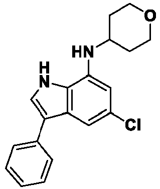 | 5-chloro-3-phenyl-N-(tetrahydro-2H-pyran-4-yl)-1H-indol-7-amine |
| 13 | 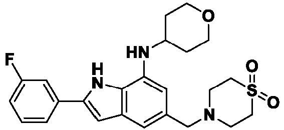 | 4-((2-(3-fluorophenyl)-7-((tetrahydro-2H-pyran-4-yl)amino)-1H-indol-5-yl)methyl)thiomorpholine 1,1-dioxide |
| 14 | 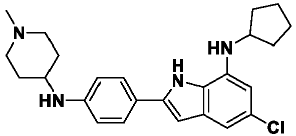 | 5-chloro-N-cyclopentyl-2-(4-((1-methylpiperidin-4-yl)amino)phenyl)-1H-indol-7-amine |
| 15 | 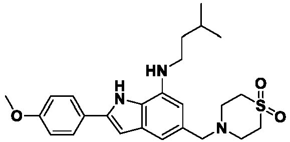 | 4-((7-(isopentylamino)-2-(4-methoxyphenyl)-1H-indol-5-yl)methyl)thiomorpholine 1,1 -dioxide |
| 16 | 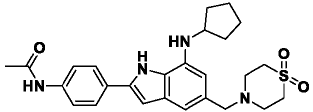 | N-(4-(7-(cyclopenylamino)-5-((1,1-dioxidothiomorpholino)methyl)-1H-indol-2-yl)phenyl)acetamide |
| 17 | 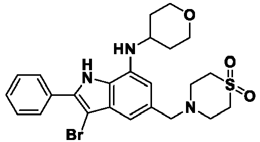 | 4-((3-bromo-2-phenyl-7-((tetrahydro-2H-pyran-4-yl)amino)-1H-indol-5-yl)methyl)thiomorpholine 1,1-dioxide |
| 18 | 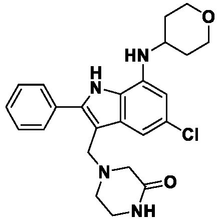 | 4-((5-chloro-2-phenyl-7-((tetrahydro-2H-pyran-4-yl)amino)-1H-indol-3-yl)methyl)piperazin-2-one |
| 19 | 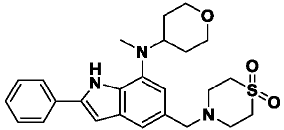 | 4-((7-(methyl(tetrahydro-2H-pyran-4-yl)amino)-2-phenyl-1H-indol-5-yl)methyl)thiomorpholine 1,1-dioxide |
| 20 | 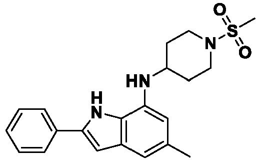 | 5-methyl-N-(1-(methylsulfonyl)piperidin-4-yl)-2-phenyl-1H-indol-7-amine |
| 21 | 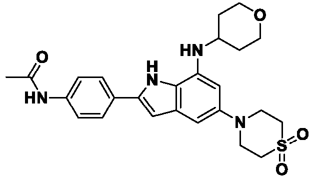 | N-(4-(5-(1,1-dioxidothiomorpholino)-7-((tetrahydro-2H-pyran-4-yl)amino)-1H-indol-2-yl)phenyl)acetamide |
| 22 | 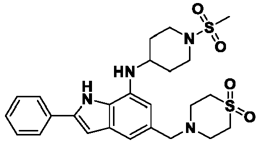 | 4-((7-((1-(methylsulfonyl)piperidin-4-yl)amino)-2-phenyl-1H-indol-5-yl)methyl)thiomorpholine 1,1-dioxide |
| 23 | 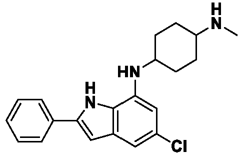 | N¹-(5-chloro-2-phenyl-1H-indol-7-yl)-N⁴-methylcyclohexane-1,4-diamine |
| 24 | 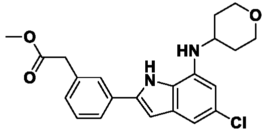 | methyl 2-(3-(5-chloro-7-((tetrahydro-2H-pyran-4-yl)amino)-1H-indol-2-yl)phenyl)acetate |
| 25 | 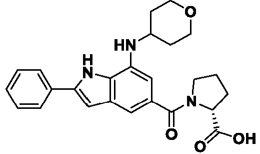 | (2-phenyl-7-((tetrahydro-2H-pyran-4-yl)amino)-1H-indol-5-carbonyl)-D-proline |
| 26 | 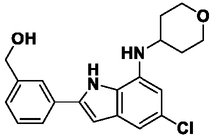 | (3-(5-chloro-7-((tetrahydro-2H-pyran-4-yl)amino)-1H-indol-2-yl)phenyl)methanol |
| 27 | 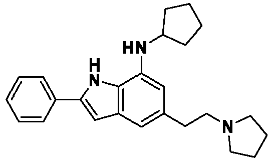 | N-eyelopentyl-2-phenyl-5-(2-(pyrrolidin-1-yl)ethyl)-1H-indol-7-amine |
| 28 | 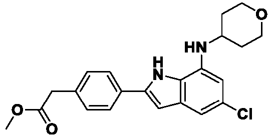 | methyl 2-(4-(5-chloro-7-((tetrahydro-2H-pyran-4-yl)amino)-1H-indol-2-yl)phenyl)acetate |
| 29 | 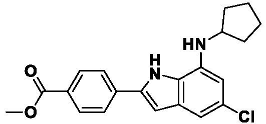 | methyl 4-(5-chloro-7-(cyclopenylamino)-1H-indol-2-yl)benzoate |
| 30 | 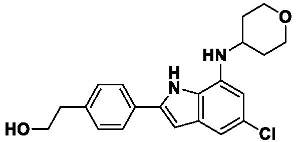 | 2-(4-(5-chloro-7-((tetrahydro-2H-pyran-4-yl)amino)-1H-indol-2-yl)phenyl)ethan-1-ol |
| 31 | 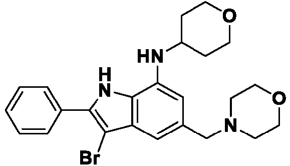 | 3-bromo-5-(morpholinomethyl)-2-phenyl-N-(tetrahydro-2H-pyran-4-yl)-1H-indol-7-amine |
| 32 | 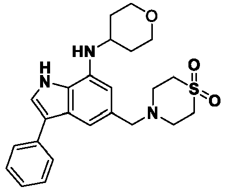 | 4-((3-phenyl-7-((tetrahydro-2H-pyran-4-yl)amino)-1H-indol-5-yl)methyl)thiomorpholine 1,1-dioxide |

In one embodiment of the present invention, the compound of Chemical Formula 1 may be a compound of Chemical Formula 2.

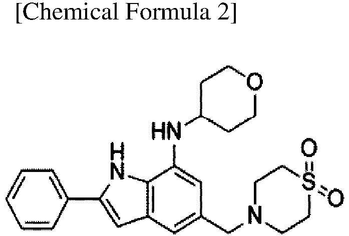

The term "anti-ovarian aging" used herein may refer to restoring ovaries whose function has been lost due to ovarian aging to normal ovaries or a similar level, or may encompass suppressing the progression of ovarian aging.

The term "ovarian follicle" used herein may refer to a cellular aggregation found in ovaries, which is a place where female hormones are produced and secreted and contains an oocyte.

The term "folliculogenesis" used herein refers to a process of maturing ovarian follicles in ovaries, containing (immature) oocytes, or any stage thereof, which includes any stage of the progression from primordial follicles to preovulatory follicles, and/or the progression from immature oocytes to egg cells.

The term "oocyte" used herein refers to a cell that can mature into eggs through meiosis.

The term "cumulus cell" refers to a granular cell that surrounds and nourishes oocytes.

The term "aging" used herein refers to the manifestation of a natural aging phenomenon in which function deteriorates over time or due to internal/external irritants (e.g., inflammation).

The term "ovarian function" is a generic term for, for example, the production of reproductive hormones, maintenance of appropriate levels of follicle-stimulating hormone or follicle production, the ovulation of ovarian follicles, luteinization, maintenance of appropriate levels of ovarian follicles and oocytes, and the histology of ovaries (e.g., size and tissue health).

The term "loss of ovarian function" used herein may refer to the loss or decline in ovarian function, which is known in the art, such as the loss of ovarian functions for pregnancy such as ovulation, implantation, fertilization, etc., or a decline in reproductive hormones secreted from the ovaries.

The term "restoration of ovarian function" used herein may refer to restoring ovarian function damaged by external factors or internal factors (particularly, aging), and the degree of recovery may be the degree to the ovaries were before being damaged due to aging or other causes or a similar degree thereto, or may indicate the degree of improvement compared to that before administration of a treatment or treatment. Here, the restoration of damaged ovarian function may refer to restoring approximately 30%, 40%, or 50% or more of normal ovarian function.

The "anti-ovarian aging" used herein not only may delay ovarian aging, but may also restore the function of aged ovaries, prolong reproductive potential or ovarian function, or alleviating ovarian inflammation, and may refer to treating ovarian aging taken as a whole.

The aged ovaries may exhibit several phenotypic defects, such as oxidative stress, the development of ovarian inflammation, decreased steroidogenesis, hair follicle production, and may lose reproductivity due to the decreased number of ovarian follicles and poor egg quality.

In one embodiment of the present invention, the anti-ovarian aging may be improving or restoring the function of aged ovaries, which have the above problems.

In one embodiment of the present invention, it was confirmed that the total number of ovarian follicles, including preantral follicles and antral follicles, increases, the development of ovarian follicles is improved, the number of oocytes increases, and the rate of preimplantation embryo development increases by the treatment with the compound of Chemical Formula 1.

Accordingly, in the present invention, the anti-ovarian aging may include one or more selected from the group consisting of an increased number of ovarian follicles, an increased number of oocytes, and an increased rate of preimplantation embryo development.

In one embodiment of the present invention, the compound of Chemical Formula 1 may increase the expression of one or more genes selected from the group consisting of longevity-related genes, steroidogenesis-related genes, primordial follicle factors, and function factors of ovary in cells.

The primordial follicle factors may enhance the survival of primordial follicles, and may regulate differentiation to granulosa cells due to the proliferation of stromal cells and development of preantral follicles, and may be genes related to follicular development, and may be one or more selected from the group consisting of Nanog, Oct3/4, and p63.

The longevity-related genes may be genes associated with prolonged survival of ovarian-related cells, and may be one or more selected from the group consisting of SOD2 and SIRT1.

The steroidogenesis-related genes may be one or more selected from the group consisting of Edn2, Tbxa2r, Oxtr, and Adrald.

The ovarian function factors may be one or more selected from the group consisting of Lhcgr, AMH, GDF9, BMP15, and Kitl.

According to the following examples, it was confirmed that the expression levels of the corresponding genes increase through treatment with the compound of Chemical Formula 1.

The compound of Chemical Formula 1 according to the present invention may be used in preventing, improving, or treating a disease related to the aging or dysfunction of ovaries by suppressing folliculogenesis and ovarian aging, or restoring the function of aged ovaries.

Therefore, the present invention provides a pharmaceutical composition for preventing or treating a disease related to the aging or dysfunction of ovaries, which includes the compound of Chemical Formula 1 or a pharmaceutically acceptable salt thereof:

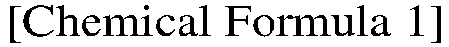

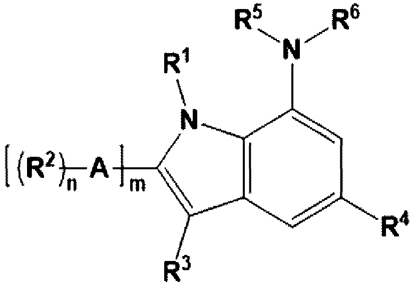

In this formula, R¹ to R⁶, A, n, and m are each the same as defined above.

In one embodiment of the present invention, the disease related to ovarian aging or dysfunction may be one or more selected from the group consisting of premature ovarian failure, sterility, infertility, miscarriages, ovarian cysts, ovarian teratomas, ovarian endometriomas, polycystic ovary syndrome, menopause, menopausal symptoms, ovarian cancer, and oophoritis, but the present invention is not limited thereto, and may be any disease that occurs due to the aging or dysfunction of ovaries without limitation.

The term "menopausal symptoms" refers to symptoms and conditions that occur in women before, during and after menopause by ovarian aging, hormonal changes, and/or other biological processes.

The menopausal symptoms may include, for example, hot flashes, sweating, sweating during sleep, sleep disorders, skin dryness, colpoxerosis, vaginal atrophy, atrophy of the lower urinary tract, dyspareunia, vaginitis, cystitis, urodynia, urinary urgency, attention problems, memory problems, anxiety, depression, fatigue, nervousness, decreased libido, muscle pain, joint pain, or osteoporosis, but the present invention is not limited thereto.

The "premature ovarian failure" refers to the disruption of ovarian function before the age of 40. The causes of premature ovarian failure include chemotherapy, radiotherapy, autoimmune disease, thyroid disease, diabetes, and surgically induced menopause (e.g., hysterectomy or oophorectomy), but the present invention is not limited thereto.

The present invention provides a pharmaceutical composition for delaying menopause, or preventing or suppressing early menopause, which includes the compound of Chemical Formula 1 or a pharmaceutically acceptable salt thereof.

The compound of Chemical Formula 1 or a pharmaceutically acceptable salt thereof according to the present invention may also be used to protect ovaries.

The "ovarian protection" may refer to protection against any injury or trauma (e.g., engraftment or injury) to ovaries. The "ovarian protection" refers to the protection of ovarian function to appropriate levels. As the aging of ovaries progresses, the possibility of injury or trauma increases, and thus the ovaries may be protected from injury or trauma by treatment with the compound of Chemical Formula 1.

The present invention provides a method for anti-ovarian aging, which includes administering the compound of Chemical Formula 1 or a pharmaceutically acceptable salt thereof to a subject in need thereof.

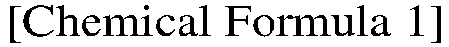

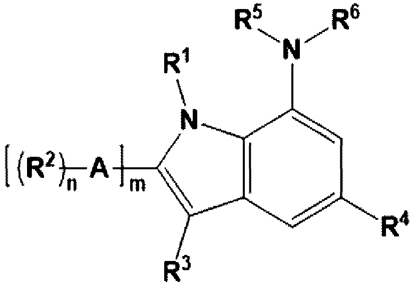

In this formula, R¹ to R⁶, A, n, and m are each the same as defined above.

In the method for anti-ovarian aging, the ovaries may be aged ovaries, and the method may include restoring the function of aged ovaries by treating the aged ovaries with the compound of Chemical Formula 1.

In addition, the present invention may provide a method of preventing or treating ovarian aging or dysfunction-related diseases, which includes administering the compound of Chemical Formula 1 to an individual in need thereof.

In the above method for anti-ovarian aging, and method of preventing or treating ovarian aging or dysfunction-related diseases, unless defined otherwise, the contents of the pharmaceutical composition for anti-ovarian aging may be applied mutatis mutandis.

In addition, the present invention may provide a use of the compound of Chemical Formula 1 or a pharmaceutically acceptable salt thereof for anti-ovarian aging, and a use of the compound of Chemical Formula 1 or a pharmaceutically acceptable salt thereof for the prevention or treatment of ovarian aging or dysfunction-related diseases.

For the above use, unless otherwise defined, the contents of the pharmaceutical composition for anti-ovarian aging may be applied mutatis mutandis.

The "treatment" used herein refers to stopping or delaying the progression of a disease when used in subjects exhibiting symptoms of a disease, and the "prevention" used herein refers to stopping or delaying the onset of disease when used in subjects that do not exhibit symptoms but are at high risk for such disease.

The "pharmaceutical composition" used herein may include a pharmaceutically acceptable carrier as needed, along with the compound of the present invention.

The compound of Chemical Formula 1 according to the present invention may be administered in a variety of oral and parenteral dosage forms in clinical administration, and may be formulated with diluents or excipients, such as a filler, an extender, a binder, a wetting agent, a disintegrant, and a surfactant.

Solid preparations for oral administration include tablets, pills, powders, granules, capsules, and troches, and such solid preparations are prepared by mixing at least one excipient, such as starch, calcium carbonate, sucrose, lactose, or gelatin with one or more of the compounds of the present invention. In addition, in addition to simple excipients, lubricants such as magnesium stearate, talc, etc. are also used. Liquid preparations for oral administration include suspensions, liquids for internal use, emulsions, and syrups, and may further include various types of excipients, for example, a wetting agent, a sweetener, a fragrance and a preservative, other than a commonly-used simple diluent such as water, or liquid paraffin.

Formulations for parenteral administration may include sterilized aqueous solutions, non-aqueous solvents, suspensions, emulsions, freeze-dried formulations, or suppositories. As the non-aqueous solvent or suspension, propylene glycol, polyethylene glycol, a vegetable oil such as olive oil, or an injectable ester such as ethyl oleate may be used. As a suppository base, Witepsol, Tween 61, cacao butter, laurin butter, glycerol, or gelatin may be used.

In addition, an effective dose of the compound of Chemical Formula 1 of the present invention for the human body may vary depending on a patient's age, body weight, gender, administration type, health condition, and severity of a disease, and is generally approximately 0.001 to 100 mg/kg/day, and preferably 0.01 to 35 mg/kg/day. Based on an adult patient weighing 70 kg, the composition of Chemical Formula 1 may be administered at a dose of generally 0.07 to 7000 mg/day, and preferably, 0.7 to 2500 mg/day, and may be administered once or in divided portions several times a day at regular intervals depending on a doctor's or pharmacist's judgement.

The term "subject" used herein include vertebrates such as mammals including humans and livestock, and birds, in which an inflammatory disease can be alleviated, prevented, or treated by administration of a pharmaceutical composition of the present invention, but the present invention is not limited thereto.

The "administration" used herein refers to introducing a given material into a human or animal by any proper method, and the administration route of a composition for prevention or treatment according to the present invention may be oral or parenteral administration via any general route as long as the composition can reach desired tissue.

The numerical values described above in the specification should be interpreted as including the equivalent range unless specified otherwise.

Hereinafter, the present invention will be described in detail with reference to the following experimental examples. However, the following Experimental Examples are merely illustrative, and the scope of the present invention are not limited by the following Experimental Examples. In addition, these Experimental Examples are merely provided to help understand the present invention, and the scope of the present invention is not limited thereby in any way.

### Example

### Materials and preparation

As a representative example of the compound of Chemical Formula 1 in this Example, 5-[(1,1-dioxido-4-thiomorpholinyl)methyl]-2-phenyl-N-(tetrahydro-2H-pyran-4-yl)-1H-indol-7-amine (hereinafter, referred to as `Example Compound' or 'Compound 1') was used.

In this experiment, as a control, young mice (7 to 9 weeks old, n=15), and as an experimental group, old mice (55 to 60 weeks old, n=30) were prepared. In the drawing, the experimental results of the young mice are indicated as 'Young,' and the experimental results of the old mice are indicated as 'Old.'

### <Collection of modified pronuclear cells>

For *in vivo* hormone stimulation, the old mice were treated with the Example compound, and 12 hours later, 75 IU pregnant mare serum gonadotropin (PMSG: RP1782725000; BioVendor, Czech Republic) was injected into the mice. A control is a group administered only 75IU PMSG. Female mice were superovulated by injecting hormones intraperitoneally at intervals of 48 hours. The administered hormones were PMSG (7.5 IU) and human chorionic gonadotropin (hCG: 668900221; LG Chem, Seoul, Korea; 7.5 IU). After secondary hormone treatment, female mice were mated with male mice (2:1 or 1:1). Modified pronuclear cells (2PNs) were obtained 24 hours after hCG injection.

### <Collection of GV oocytes and embryos >

Female mice (young mice: 7-8 weeks old; old mice: 40 weeks or older) were superovulated by intraperitoneally injecting pregnant mare serum gonadotropin (PMSG; G4527, Sigma Aldrich; 7.5 IU). Germinal vesicle (GV) oocytes were collected from the ovaries 48 hours after PMSG injection. For zygotes, female mice were injected with PMSG, and 48 hours later, injected with hCG, and then placed overnight with BDF1 stud male mice, which were approximately 10 to 12 weeks old. The next day, plugs of the female mice were examined, and zygotes and two-cell embryos were collected from the oviducts 18 to 20 hours and 36 hours after hCG injection, respectively.

### Experimental Example 1. Confirmation of increased oocytes and development of ovarian follicles by treatment with Example Compound

In Experimental Example 1, the effect of treatment with Example Compound on mouse ovaries stimulated with PMSG *in vivo* was evaluated. Light microscopy imaging was performed on the ovaries of young mice, and old mice treated and not treated with Example Compound.

Each sample was fixed with 4% paraformaldehyde at room temperature for 30 minutes, and the fixed tissue was washed with PBS and embedded in paraffin with a thickness of 5 µm. For histological analysis through hematoxylin and eosin staining, the sample was deparaffinized in xylene from 100% to 75% per 10 minutes, rehydrated with 100%, 95%, 80% and 75% EtOH per 10 minutes at room temperature, and then subjected to immunofluorescence staining with hematoxylin and eosin. Antigens were regenerated by boiling in an autoclave container (Nalgene Jars) with 0.01M citrate buffer (pH 6.0) for 20 minutes.

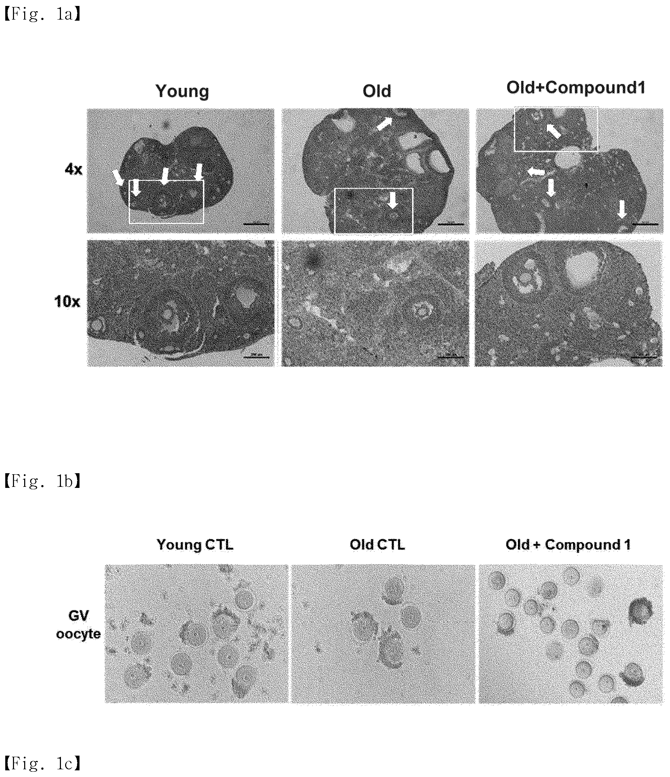

FIG. 1A shows a set of histological images of mouse ovarian tissue stained with hematoxylin and eosin after hormonal stimulation according to the presence or absence of treatment with Example Compound. It can be seen that the total number of ovarian follicles (marked with an arrow), including preantral follicles and antral follicles, is significantly higher in the ovaries treated with Example Compound, compared to the ovaries of the control.

After inducing superovulation, the numbers of GV oocytes in the ovaries of the old mice and young mice treated with Example Compound were evaluated. The results are shown in FIGS. 1B and 1C.

The measurement of the number of GV oocytes was performed by collecting mouse eggs from each group (the number of eggs of young mice: 15; the number of eggs of old mice: 30; and the number of eggs of old mice treated with Example Compound: 30), physically chopping the eggs to collect ovarian follicles, and isolating GV oocytes in the ovarian follicles to count the number of cells.

FIG. 1B shows a set of optical microscope images, FIG. 1C shows a graph of the number of GV oocytes, and in FIGS. 1B and 1C, CTL indicates a control that was not treated with Example Compound.

FIGS. 1B and 1C show that, at the GV stage, the total number of oocytes collected from the old ovaries treated with Example Compound is significantly higher than that of the old ovaries not treated with Example Compound as a control.

### Experimental Example 2. Pre-implantation embryo development according to treatment with Example Compound

In Experimental Example 2, the effect of fertilized 2PN embryo development on the ovaries of the old mice treated with Example Compound was evaluated.

27 pronuclear cells of the old mice (n=15) treated with Example Compound and 62 pronuclear cells of the old mice (n=15) not treated with Example Compound as a control were cultured. Afterward, the number of cells at each of 2-cell, 4-cell, 8-cell, morula, early blastocyst, and late blastocyst stages was measured using a microscope, and expressed as a percentage.

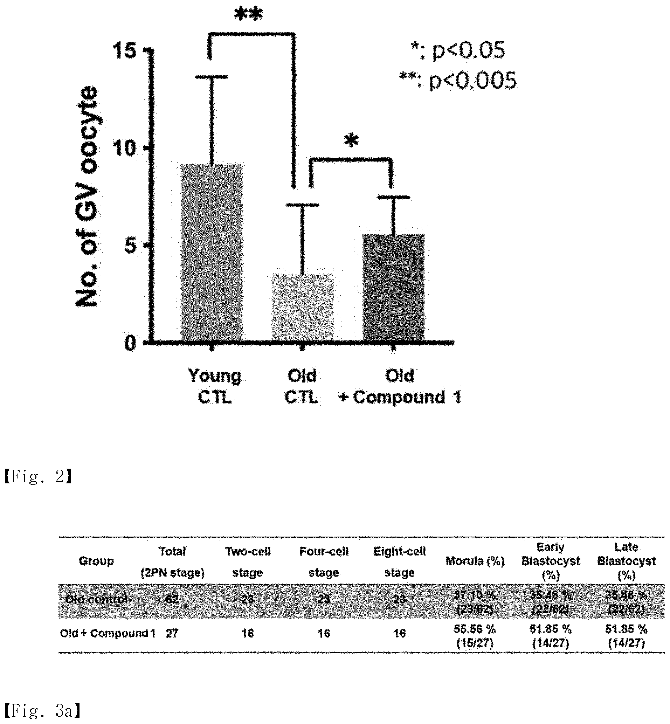

As shown in FIG. 2, the development rate of Example Compound embryo is 20% higher than that of the conventional control embryo at the morula, early blastocyst, and late blastocyst stages. Specifically, the embryo development rate of the old mice treated with Example Compound at the morula stage was 55.56%. However, the embryo development rate of the control was significantly low at 37.10%. The early blastocyst rate for the embryo treated with Example Compound (51.85%) was higher than that of the embryo of the control (35.48%), and finally, the late blastocyst rate of the embryo of the old mice treated with Example Compound (51.85%) was significantly higher than that of the embryo of the control (35.48%).

### Experimental Example 3. Confirmation of gene expression pattern in ovaries

### < Confirmation of gene expression pattern through next generation sequencing>

A major change in gene expression caused by treatment with Example Compound was confirmed through next generation sequencing (NGS).

mRNA sequencing was performed according to References [1, 2]. RNA was extracted using a TRIzol (15596026, Thermo Fisher) solution. Sequencing libraries were prepared according to the manufacturer's instructions using the TruSeq Stranded mRNA LT Sample Prep Kit (Illumina). Paired end 101bp sequencing was performed using the Illumina platform (NovaSeq 6000, Illumina). The results of two experiments were obtained for each group.

According to the criteria (1st trimming: base quality < 3, window size = 4, mean quality = 15; 2nd trimming: min length = 36bp), paired end read data was trimmed using Trimmomatic (Bolger et al., 2014). Using the HISAT2 program (Kim et al., 2019; Nat Biotechnol 37, 907-915, 2019), trimmed reads were mapped to the UCSC genome database. Also, the mapping results were quantified using StringTie (Pertea et al., 2015; Nat Biotechnol. 2015 Mar; 33(3): 290-295) and gene/transcript-based annotations (NCBI Annotation Release 108).

Differential expression gene analysis was performed using DESeq2 according to the criteria (baseMean counts > 14, false discover rate (FDR) < 0.1, and log2FoldChange > 1). Gene set enrichment analysis (GSEA) was performed using clusterProfiler packages (Reference [3]) of RBioconductor, and gene ontology (GO; biological process, cellular component, and molecular function) and canonical pathways (REACTOME, WikiPathway (Reference [4]) were analyzed.

FIG. 3A shows the results of analyzing RNAs purified from the ovaries of a young group (Young, YNG; 10 weeks; n=2), an old group (Old; 55 weeks; n=2), and an old group (Old+compound1; 55 weeks; n=2) injected with Example Compound by NGS.

In FIG. 3A, differentially expressed genes (DEGs) between groups were expressed in a Venn diagram format. It was confirmed that there are 1,214 DEGs between the old group and the young group, 1,021 DEGs between the old group and the old group treated with Example Compound, and among these DEGs, there are 267 DEGs in common. It was confirmed that there are enriched gene ontology (GO) terms, involved with the regulation of the immune system, in the common DEGs.

In addition, among GOs involved with immune system regulation, DEGs related to tumor necrosis factor superfamily cytokine production (n=38) were confirmed. Fcerlg, Slamf9, Clec4a3, Ptpn6, Tyrobp, Adipoq, Bcl3, Trem2, and Sash3 were identified as genes that were increased in the old group and down-regulated by Example Compound.

### < Confirmation of change in immune system regulatory gene expression through RT-qPCR>

In this experiment, a change in the expression of immune system regulatory genes (genes involved with tumor necrosis factor superfamily cytokine production) was quantitatively evaluated using real-time quantitative PCR (RT-qPCR) according to the treatment of aged models with Example Compound.

Based on the Kyoto Encyclopedia of Genes and Genomes (KEGG) signal pathway, primers for up-regulated and down-regulated genes were selected.

RT-qPCR was performed on each ovary using the TRIzol protocol, total RNA was extracted from a lysate using the RNeasy mini kit (Qiagen Ltd.), and the extracted total RNA was reverse-transcribed using a Superscript kit (Invitrogen) containing a random hexamer to synthesize cDNA.

Gene expression levels were confirmed using primers and the SsoAdvanced Universal SYBR Green Supermix (Bio-Rad, US) in the CFX96 Touch Real-Time PCR detection system (Bio Rad, US). Primer sequences are shown in Table 2.

**[Table 2]**

| Gene | | **Sequence (5'-3')(F-forward, R-reverse)** | |
|---|---|---|---|
| **Ptpn6** | Protein Tyrosine Phosphatase Non-Receptor Type 6 | F | GGA CTT CTA TGA CCT GTA CGG A |
| | | R | CGA GCA GTT CAG TGG GTA CTT |
| **Fcer1g** | **Fc** Epsilon Receptor 1g | F | ATC TCA GCC GTG ATC TTG TTC T |
| | | R | ACC ATA CAA AAA CAG GAC AGC AT |
| **Tyrobp** | Transmembrane Immune Signaling Adaptor TYROBP | F | CCC AAG ATG CGA CTG TTC TTC |
| | | R | GTC CCT TGA CCT CGG GAG A |
| **Slamf9** | SLAM family member 9 | F | AGA AAA GCT ATG AGA CGC C |
| | | R | AGG ACC TCA AAA GCC AAT C |
| **Clec4a3** | C-type lectin domain family 4, member a3 | F | ACT TCA ACT GAC TTG GTG G |
| | | R | AAA TCC TGT TCT TCC TGG C |

For each gene tested, a comparative threshold cycle (CT) method that normalizes the accumulated PCR product to the reference gene beta-actin was used, and repeated three times.

The PCR product was loaded on a 2% agarose gel and stained with Safeview^{™} (Applied Biological Materials, Richmond, Canada), and the gel was visualized under UV light using a gel documentation system (WSE-6100 LuminoGraph; ATTO, Tokyo, Japan), and the product bands were analyzed by the analysis of intensity ratios using ImageJ software.

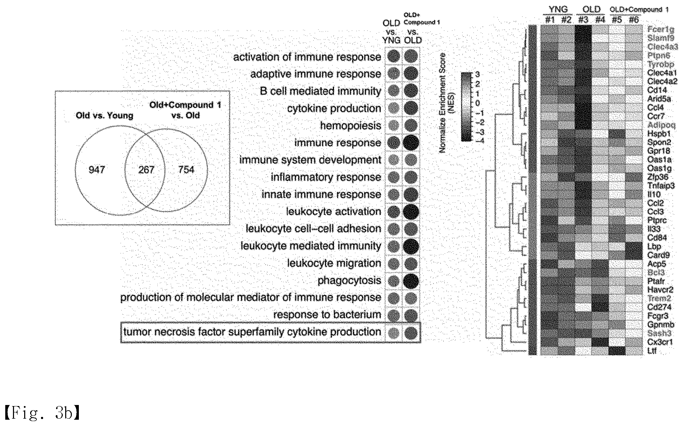

As shown in FIG. 3B, it can be confirmed that the mRNA expression levels of genes involved with tumor necrosis factor superfamily cytokine production, such as Ptpn6, Fcerlg, Tyrobp, Slamf9, and Clec4a3, significantly increased in the old control group. However, it can be confirmed that, in the old experimental group (Old) treated with Example Compound, the expression levels were lowered to levels similar to those of the young control (Young).

### Experimental Example 4. Confirmation of gene expression pattern of ovarian function

The expression of ovarian function-related genes in the ovaries of old mice treated with Example Compound was confirmed by reverse transcription-polymerase chain reaction (RT-PCR).

mRNA was extracted from oocytes using a TRIzol (TRIzol RNA, 15596026, Thermo Fisher) solution and subjected to RT-PCR. The PCR product was stained with SafeView^{™} (Applied Biological Materials, Richmond, Canada) and loaded on a 2% agarose gel. Additionally, the gel was visualized under UV light using a gel documentation system (WSE-6100 LuminoGraph; ATTO, Tokyo, Japan). The intensity ratios of the product bands were analyzed using the ImageJ software program.

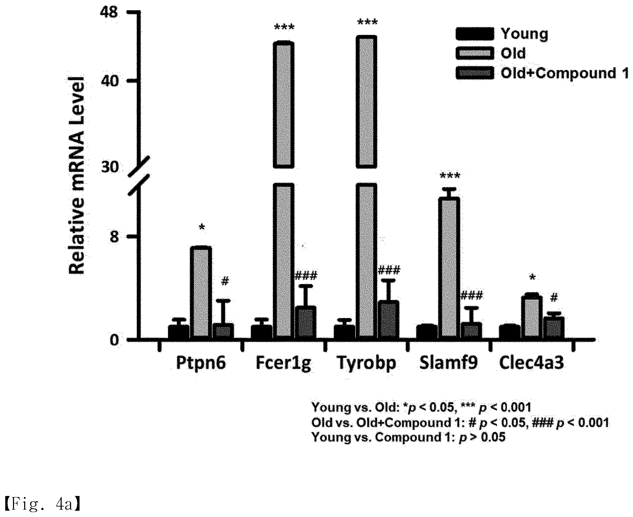

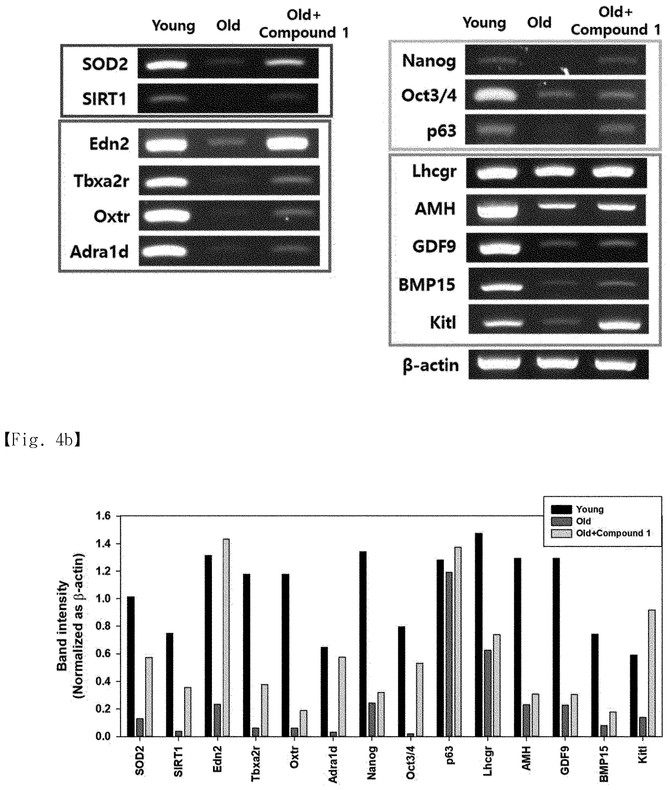

The expression of primordial follicle factors, such as Nanog, Oct3/4, and p63, and ovarian function factors, such as AMH and BMP15, in the ovaries of the mice treated with Example Compound were analyzed by RT-PCR. The results are shown in FIG. 4.

In FIGS. 4A and 4B, "Young" indicates a young mouse, "Old" indicates an old mouse, and "Old+Compound 1" indicates an old mouse treated with Example Compound.

In FIG. 4A, the upper left box indicates longevity-related genes, the lower left box indicates steroidogenesis-related genes, the upper right box indicates primordial follicle factors, and the lower right box indicates ovarian function factors. FIG. 4B is a graph of quantifying the normalization of the band image of each gene shown in FIG. 4A to beta-actin.

As shown in FIGS. 4A and 4B, the mRNA expression of Oct3/4, Nanog, p63, Lhcgr, AMH, GDF9, BMP15, and Kitl was significantly higher in the experimental group treated with Example Compound, compared to the control not treated with Example Compound.

In addition, as shown in FIGS. 4A and 4B, as a result of analyzing the longevity-related genes such as SOD2 and SIRT1, it was confirmed that, in the old mice treated with Example Compound, the expression levels of SOD2 and SIRT1 were higher compared to the old mice not treated with Example Compound. In addition, in the old mice treated with Example Compound, the expression levels of the steroid production-related genes such as Edn2, Tbxa2r, Oxtr, and Adrald significantly increased, compared to the old mice not treated with Example Compound.

### [References]

[1] Jeffrey A Martin and Zhong Wang (2011) Next-generation transcriptome assembly. Nat Rev Genet. 12(10):671-82.
[2] Love MI, Huber W, Anders S. (2014) Moderated estimation of fold change and dispersion for RNA-seq data with DESeq2. Genome Biol 15:550.
[3] Subramanian A, et al. (2005) Gene set enrichment analysis: a knowledge-based approach for interpreting genome-wide expression profiles. Proc Natl Acad Sci U S A 102:15545-15550.
[4] Jassal B, et al. (2020) The reactome pathway knowledgebase. Nucleic Acids Res 48:D498-d503.

## Claims

1. A pharmaceutical composition for anti-ovarian aging, comprising:
a compound of Chemical Formula 1 or a pharmaceutically acceptable salt thereof as an active ingredient: 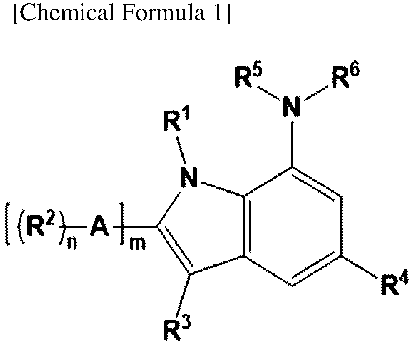 In this formula,
n is an integer from 1 to 3,
m is 0 or 1,
A is phenyl,
R¹ is hydrogen or C₁-C₆ alkyl,
R² is hydrogen, halogen, C₁-C₆ alkoxy, -C₁₋C₆ alkylene-OH, -(CH₂)ₚCO₂R⁷, -NHR⁸, - N(H)S(O)₂R⁷, or -NHC(O)R⁷, wherein p is an integer from 0 to 3, R⁷ is hydrogen or C₁-C₃ alkyl, and R⁸ is C₁-C₃ alkylpiperidinyl or C₁-C₃ alkylsulfonyl,
R³ is hydrogen, halogen, C₁-C₆ alkyl, phenyl, or -(CH₂)ₚ-heterocycle, in which the heterocycle is a 5- or 6-membered ring including 1 or 2 hetero atoms selected from S, N, and O atoms, wherein p is an integer from 0 to 3, provided that when m is 0, R³ is phenyl,
R⁴ is halogen, C₁-C₆ alkyl, -C₁₋C₆ alkylene-OH, -O-phenyl, -(CH₂)ₚCO₂R⁷, -(CH₂)ₚ-heterocycle, in which the heterocycle is a 5- or 6-membered ring including 1 or 2 hetero atoms selected from S, N, and O atoms, or proline-N-carbonyl wherein p is an integer from 0 to 3, and R⁷ is the same as defined above,
R⁵ is hydrogen or C₁-C₆ alkyl,
R⁶ is C₁-C₆ alkyl, C₃-C₆ cycloalkyl, heterocycle, or -C₁-C₆ alkylene-heterocycle, in which the heterocycle is a 3- to 8-membered ring including 1 to 3 hetero atoms selected from S, N, and O atoms, and R⁶ may be substituted with C₁-C₆ alkylamine, -C₁₋C₆ alkylene-OH, or C₁-C₆ alkylsulfonyl.

2. The pharmaceutical composition of claim 1, wherein R³ is hydrogen, halogen, phenyl, or -(CH₂)ₚ-heterocycle in which the heterocycle is morpholino or piperazinonyl, and p is an integer of 0 or 1, provided that when m is 0, R³ is phenyl,
R⁴ is halogen, C₁-C₃ alkyl, -C₁₋C₃ alkylene-OH, -O-phenyl, -(CH₂)ₚCO₂-ethyl, -(CH₂)ₚ-heterocycle in which the heterocycle is thiomorpholino, morpholino, piperazinonyl, or pyrrolidinyl, or proline-N-carbonyl, wherein p is an integer of 0 or 1,
R⁵ is hydrogen or C₁-C₃ alkyl,
R⁶ is C₁-C₃ alkyl, C₃-C₆ cycloalkyl, heterocycle, or -C₁-C₃ alkylene-heterocycle, wherein the heterocycle is tetrahydro-2H-pyran, or piperidinyl, and when R⁶ is heterocycle or - C₁-C₃ alkylene-heterocycle, the heterocycle may be substituted with C₁-C₆ alkylamine, -C₁₋C₆ alkylene-OH, or C₁-C₆ alkylsulfonyl.

3. The pharmaceutical composition of claim 1, wherein the compound of Chemical Formula 1 is any one selected from the group consisting of the following compounds:
<1> 5-[(1,1-dioxido-4-thiomorpholinyl)methyl]-2-phenyl-N-(tetrahydro-2H-pyran-4-yl)-1H-indol-7-amine; <2> ethyl 7-(cyclopentylamino)-2-phenyl-1H-indol-5-carboxylate; <3> (7-(cyclopentylamino)-2-phenyl-1H-indol-5-yl)methanol; <4>5-chloro-N,1-dimethyl-2-phenyl-N-(tetrahydro-2H-pyran-4-yl)-1H-indol-7-amine; <5> 4-((7-(cyclopentylamino)-2-(3-fluorophenyl)-1H-indol-5-yl)methyl)piperazin-2-one; <6> 4-((2-phenyl-7-(((tetrahydro-2H-pyran-4-yl)methyl)amino)-1H-indol-5-yl)methyl)thiomorpholine 1,1-dioxide; <7> 5-chloro-N-(1-methylpiperidin-4-yl)-2-phenyl-1H-indol-7-amine; <8> 5-phenoxy-2-phenyl-N-(tetrahydro-2H-pyran-4-yl)-1H-indol-7-amine; <9> 5-chloro-3-(morpholinomethyl)-2-phenyl-N-(tetrahydro-2H-pyran-4-yl)-1H-indol-7-amine; <10> 2-(4-((5-fluoro-2-phenyl-1H-indol-7-yl)amino)piperidin-1-yl)ethan-1-ol; <11> N-(4-(5-chloro-7-(cyclopentylamino)-1H-indol-2-yl)phenyl)methanesulfonamide; <12> 5-chloro-3-phenyl-N-(tetrahydro-2H-pyran-4-yl)-1H-indol-7-amine; <13> 4-((2-(3-fluorophenyl)-7-((tetrahydro-2H-pyran-4-yl)amino)-1H-indol-5-yl)methyl)thiomorpholine 1,1-dioxide; <14>5-chloro-N-cyclopentyl-2-(4-((1-methylpiperidin-4-yl)amino)phenyl)-1H-indol-7-amine; <15> 4-((7-(isopentylamino)-2-(4-methoxyphenyl)-1H-indol-5-yl)methyl)thiomorpholine 1,1-dioxide; <16> N-(4-(7-(cyclopentylamino)-5-((1,1-dioxidothiomorpholino)methyl)-1H-indol-2-yl)phenyl)acetamide; <17> 4-((3-bromo-2-phenyl-7-((tetrahydro-2H-pyran-4-yl)amino)-1H-indol-5-yl)methyl)thiomorpholine 1,1-dioxide; <18> 4-((5-chloro-2-phenyl-7-((tetrahydro-2H-pyran-4-yl)amino)-1H-indol-3-yl)methyl)piperazin-2-one; <19> 4-((7-(methyl(tetrahydro-2H-pyran-4-yl)amino)-2-phenyl-1H-indol-5-yl)methyl)thiomorpholine 1,1-dioxide; <20> 5-methyl-N-(1-(methylsulfonyl)piperidin-4-yl)-2-phenyl-1H-indol-7-amine; <21> N-(4-(5-(1,1-dioxidothiomorpholino)-7-((tetrahydro-2H-pyran-4-yl)amino)-1H-indol-2-yl)phenyl)acetamide; <22> 4-((7-((1-(methylsulfonyl)piperidin-4-yl)amino)-2-phenyl-1H-indol-5-yl)methyl)thiomorpholine 1,1-dioxide; <23> N¹-(5-chloro-2-phenyl-1H-indol-7-yl)-N⁴-methylcyclohexane-1,4-diamine; <24> methyl 2-(3-(5-chloro-7-((tetrahydro-2H-pyran-4-yl)amino)-1H-indol-2-yl)phenyl)acetate; <25> (2-phenyl-7-((tetrahydro-2H-pyran-4-yl)amino)-1H-indol-5-carbonyl)-D-proline; <26> (3-(5-chloro-7-((tetrahydro-2H-pyran-4-yl)amino)-1H-indol-2-yl)phenyl)methanol; <27> N-cyclopentyl-2-phenyl-5-(2-(pyrrolidin-1-yl)ethyl)-1H-indol-7-amine; <28> methyl 2-(4-(5-chloro-7-((tetrahydro-2H-pyran-4-yl)amino)-1H-indol-2-yl)phenyl)acetate; <29> methyl 4-(5-chloro-7-(cyclopentylamino)-1H-indol-2-yl)benzoate; <30> 2-(4-(5-chloro-7-((tetrahydro-2H-pyran-4-yl)amino)-1H-indol-2-yl)phenyl)ethan-1-ol; <31> 3-bromo-5-(morpholinomethyl)-2-phenyl-N-(tetrahydro-2H-pyran-4-yl)-1H-indol-7-amine; and <32> 4-((3-phenyl-7-((tetrahydro-2H-pyran-4-yl)amino)-1H-indol-5-yl)methyl)thiomorpholine 1,1-dioxide.

4. The pharmaceutical composition of claim 1, wherein the compound of Chemical Formula 1 is a compound of Chemical Formula 2 below,

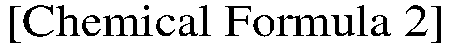

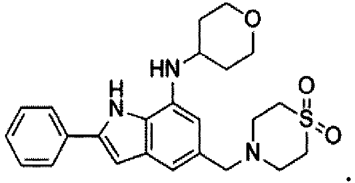

5. The pharmaceutical composition of claim 1, wherein the anti-ovarian aging is restoring the function of aged ovaries.

6. The pharmaceutical composition of claim 1, wherein the anti-ovarian aging includes one or more selected from the group consisting of an increased number of ovarian follicles, an increased number of oocytes, and an increased rate of preimplantation embryo development.

7. The pharmaceutical composition of claim 1, wherein the compound of Chemical Formula 1 increases the expression of one or more genes selected from the group consisting of longevity-related genes such as SOD2 and SIRT1; steroidogenesis-related genes such as Edn2, Tbxa2r, Oxtr, and Adrald; primordial follicle factors such as Nanog, Oct3/4, and p63; and ovarian function factors such as Lhcgr, AMH, GDF9, BMP15, and Kitl in cells.

8. A pharmaceutical composition for preventing or treating a disease related to the aging or dysfunction of ovaries, comprising:
a compound of Chemical Formula 1 or a pharmaceutically acceptable salt thereof as an active ingredient: 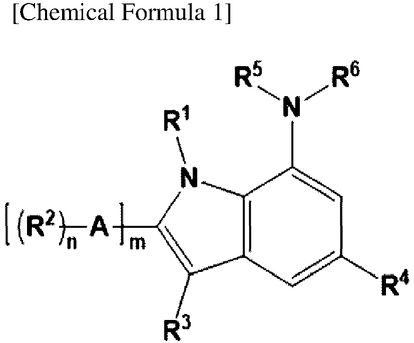 In this formula, R¹ to R⁶, A, n, and m are each the same as defined in claim 1.

9. The pharmaceutical composition of claim 8, wherein the disease related to the aging or dysfunction of ovaries includes one or more selected from the group consisting of premature ovarian failure, sterility, infertility, miscarriages, ovarian cysts, ovarian teratomas, ovarian endometriomas, polycystic ovary syndrome, menopause, menopausal symptoms, ovarian cancer, and oophoritis.

10. A method for anti-ovarian aging, comprising:
administering a compound of Chemical Formula 1 or a pharmaceutically acceptable salt thereof into a subject in need thereof: 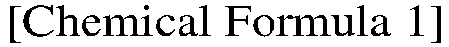 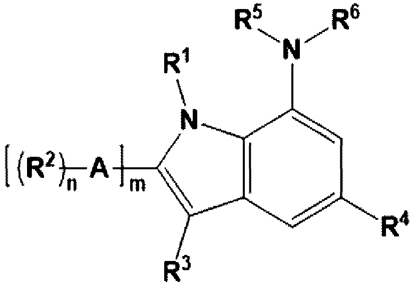 In this formula, R¹ to R⁶, A, n, and m are each the same as defined in claim 1.
